# EUROPEAN PATENT APPLICATION

(11) **EP 3 029 024 A1**
(43) Date of publication of application: **08.06.2016**
(21) Application number: 14382489.4
(22) Date of filing: 02.12.2014
(51) Int. Cl.: C07C 217/88, A61K 31/136, A61P 25/00

(54) **2-(2-aminophenoxy)-3-chloronaphthalene-1,4-dione compounds having orexin 2 receptor agonist activity**

(71) Applicant: Ferrer Internacional, S.A., 08029 Barcelona (ES)
(72) Inventor: Cano, Montserrat, E-08691 Monistrol (ES); Grima Poveda, Pedro Manuel, E-08028 Barcelona (ES); Palomer Benet, Albert, E-08014 Barcelona (ES)
(74) Representative: ABG Patentes, S.L.

(57) **Abstract**

The invention provides a compound of formula (**I**) 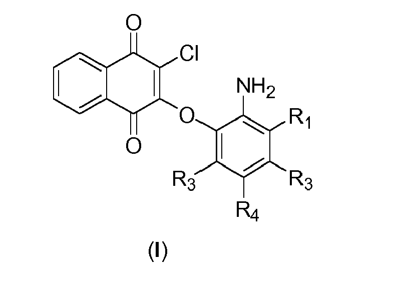 wherein R₁-R₄ represent different meanings, and pharmaceutically acceptable salts thereof. The invention also provides synthetic procedures for preparing the compounds of formula (I), pharmaceutical compositions comprising them, their use as medicaments, their use as therapeutic active substances for the treatment of conditions mediated by agonizing the orexin 2 receptor, and their use as therapeutic active substances for the treatment of narcolepsy, excessive daytime sleepiness, nighttime insomnia, depression, sleep apnea and jet lag, for promoting wakefulness and emergence from anesthesia, and for preventing diet-induced obesity.

## Description

### Field of the art

The invention relates to novel 2-(2-aminophenoxy)-3-chloronaphthalene-1,4-dione compounds having orexin 2 receptor (OX2R) agonist activity. The invention also relates to their manufacture, pharmaceutical compositions comprising them, and their use as medicaments.

### State of the art

Narcolepsy is a condition characterized by recurrent episodes of daytime somnolence and lapses in consciousness (microsomnias) that may be associated with automatic behaviors and amnesia. Cataplexy (sudden bilateral loss of muscle tone), sleep paralysis, and hypnagogic hallucinations (dreams that break off on wakefulness, which can cause visual, auditory, or touchable sensations) frequently accompany narcolepsy. The pathophysiology of narcolepsy includes deregulation of rapid eye movement (REM) sleep-onset. This "sleep-onset REM period" is regarded as the diagnostic indication for narcolepsy (Nuñez A. et al., Hypocretin/Orexin Neuropeptides: Participation in the Control of Sleep-Wakefulness Cycle and Energy Homeostasis, Curr. Neuropharmacol., 2009, 7(1), 50-59).

Narcolepsy is typically treated with amphetamine-like stimulants (sleepiness) and antidepressants (cataplexy). Newer compounds, such as modafinil (non-amphetamine wake-promoting compound for excessive daytime sleepiness) and sodium oxybate (short-acting sedative for fragmented nighttime sleep, cataplexy, excessive daytime sleepiness), are increasingly used. Recent discoveries indicate that the major pathophysiology of human narcolepsy is the loss of neurons located in the lateral hypothalamus that produce the neuropeptide hypocretin (orexin). Approximately 90% of people diagnosed as having narcolepsy with cataplexy are hypocretin ligand deficient. (Zeitzer J. M. et al., The neurobiology of hypocretins (orexins), narcolepsy and related therapeutic interventions, Trends Pharmacol. Sci., 2006 Jul.; 27(7):368-74).

Orexin agonists stabilize wake by acting directly on orexin deficiency instead on just partially treating the symptoms (modafinil and sodium oxybate), so the OX2R agonists represent the first truly replacement therapy for narcolepsy.

The orexin-2/hypocretin-2 receptor gene is mutated in canine narcolepsy and disruption of the prepro-orexin/hypocretin ligand gene results in both an animal model of narcolepsy and sporadic cases of the human disease. This evidence suggests that the structure of the OX2R gene, and its homologue, the OX1R gene, both members of the G protein-coupled receptor (GPCR) family, and the gene encoding the peptide ligands, the prepro-orexin/hypocretin gene, may be variables in the etiology of sleep disorders (Thompson, M. D., et al., Variants of the orexin2/hcrt2 receptor gene identified in patients with excessive daytime sleepiness and patients with Tourette's syndrome comorbidity, Am. J. Med. Genet., (2004), 129B:69-75).

Kelz et al. (An essential role for orexins in emergence from general anesthesia, Proc. Natl. Acad. Sci. USA, 2008 Jan 29; 105(4), 1309-14) demonstrated that the genetic ablation of orexinergic neurons, which causes acquired murine narcolepsy, delays emergence from anesthesia, and confirmed that the endogenous orexin system promotes wakefulness, thus facilitating emergence from anesthesia.

Orexins excite the cerebellar interpositus nucleus neurons through OX2R and suggest that the central orexinergic nervous system may actively participate in motor control through its modulation on one of the final outputs of the spinocerebellum. (Yu L. et al., Orexins excite neurons of the rat cerebellar nucleus interpositus via orexin 2 receptors in vitro, Cerebellum, (2010) Mar.; 9(1), 88-95).

Given that the OX2R agonists can be used in the treatment of narcolepsy and excessive daytime sleepiness, beneficial effects in derived or related conditions such as nighttime insomnia, depression, sleep apnea and jet lag are to be expected.

Funato H. et al. (Enhanced Orexin Receptor-2 Signaling Prevents Diet-Induced Obesity and Improves Leptin Sensitivity, Cell Metab., 2009 January 7; 9(1), 64-76) demonstrated that the prolonged central administration of an OX2R-selective peptide agonist inhibits diet-induced obesity, and concluded that enhanced orexin-OX2R signaling confers resistance to diet-induced features of the metabolic syndrome through negative energy homeostasis and improved leptin sensitivity.

The most of orexin 2 receptor agonists known to date, such as BLX-1026 (Mukherjee A. et al., In-vitro metabolism studies with BLX-1026, a novel orexin-2 agonist with anti-obesity activity, The FASEB Journal, 2008, 22, 920.2.), and those reported by Asahi S. et al. (Development of an orexin-2 receptor selective agonist, [Ala11, d-Leu15] orexin-B, Bioorganic & Medicinal Chemistry Letters, 13 (2003), 111-3) and Lee J. et al. (Discovery of an orexin receptor positive potentiator, Chemical Science, (2010), 1(1), 48-54) are aminoacid derivatives. Non-aminoacid orexin 2 receptor agonists were firstly disclosed in US8258163B2, and are cyclic guanidinyl compounds. However, there is a need for alternative compounds active as orexin 2 receptor agonists, being suitable for the treatment of narcolepsy. Such compounds would have an appropriate pharmacological profile, an acceptable side effects profile, and a low potential for interaction with co-administered medicaments, given that these compounds may be used for the treatment of chronic diseases, such as narcolepsy and related disorders.

The research of new orexin 2 receptor agonists that may be useful in the treatment of narcolepsy and excessive daytime sleepiness responds to a fundamental health need, and therefore justifies continued research for compounds with improved properties.

The compounds of the present invention share structural similarities with those described in the co-pending unpublished application PCT/EP2014/062349. However, the compounds of the present invention are more potent agonists of the orexin 2 receptor (OX2R) than the compounds described in PCT/EP2014/062349.

As a result, the compounds of the present invention are particularly useful in the treatment and prevention of all those conditions that are mediated by agonizing the orexin 2 receptor (OX2R). Some non-limiting examples of these conditions are narcolepsy, excessive daytime sleepiness, and nighttime insomnia, depression, sleep apnea, jet lag and diet-induced obesity. Likewise the compounds of the present invention are useful for promoting wakefulness and emergence from anesthesia.

### Summary of the invention

The invention provides a compound of formula (I) 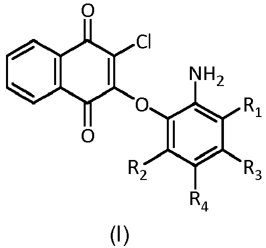 and pharmaceutically acceptable salts thereof.

It is another object of this invention to provide synthetic procedures for preparing the compounds of formula (I), pharmaceutical compositions comprising them, their use as medicaments, their use as therapeutic active substances for the treatment of conditions mediated by agonizing the orexin 2 receptor, and their use as therapeutic active substances for the treatment of narcolepsy, excessive daytime sleepiness, nighttime insomnia, depression, sleep apnea and jet lag, for promoting wakefulness and emergence from anesthesia, and for preventing diet-induced obesity.

### Detailed description of the invention

### Definitions

When describing the compounds, processes, compositions and uses of the invention, the following terms have the following meanings, unless otherwise indicated.

The term "linear or branched C₁₋₃ alkyl" as used herein refers to a monovalent saturated hydrocarbon group which may be linear or branched and has from 1 to 3 carbon atoms. Representative alkyl groups include, by way of example, methyl, ethyl, n-propyl (n-Pr) and isopropyl (i-Pr), and the like.

The term "linear or branched C₁₋₃ alkoxy" as used herein refers to a monovalent linear or branched C₁₋₃ alkyl group linked to an oxygen (ether) atom. Representative alkyl groups include, by way of example, methoxy, ethoxy, n-propoxy and isopropoxy, and the like.

The term "excipient" as used herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The term "medicament" as used herein means a pharmaceutical composition suitable for administration of the pharmaceutically active compound to a patient.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The term "condition" as used herein refers to a disease state for which the compounds and salts of the present invention are being used against.

The term "conditions mediated by agonizing the orexin 2 receptor" as used herein includes, in a non-limitative manner, narcolepsy, excessive daytime sleepiness, nighttime insomnia, depression, sleep apnea, jet lag and diet-induced obesity.

In a first aspect, the invention provides a compound of formula (I) or a pharmaceutically acceptable salts thereof 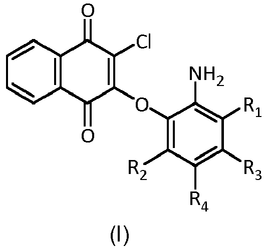 wherein
R₁ is selected from the group consisting of hydrogen, chlorine, bromine and linear or branched C₁₋₃ alkyl and R₂ is selected from the group consisting of hydrogen, chlorine and linear or branched C₁₋₃ alkyl with the condition that at least one of R₁ and R₂ is different from hydrogen;
R₃ is selected from the group consisting of hydrogen, halogen and linear or branched C₁₋₃ alkyl and R₄ is selected from the group consisting of hydrogen, chlorine, bromine, linear or branched C₁₋₃ alkoxy and linear or branched C₁₋₃ alkyl with the condition that at least one of R₃ and R₄ is different from hydrogen;
with the proviso that R₂ and R₃ are not simultaneously a chlorine atom
and pharmaceutically acceptable salts thereof.

In a preferred embodiment the invention provides a compound of formula (I) or a pharmaceutically acceptable salts thereof 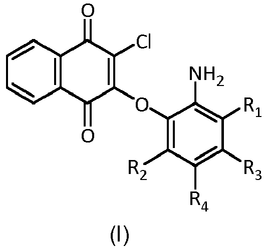 wherein
R₁ is selected from the group consisting of hydrogen and methyl and R₂ is selected from the group consisting of hydrogen, chlorine and methyl with the condition that at least one of R₁ and R₂ is different from hydrogen;
R₃ is selected from the group consisting of hydrogen, bromine, chlorine and methyl and R₄ is selected from the group consisting of hydrogen, methoxy and methyl with the condition that at least one of R₃ and R₄ is different from hydrogen;
with the proviso that R₂ and R₃ are not simultaneously a chlorine atom
and pharmaceutically acceptable salts thereof.

In a preferred embodiment R₃ is selected from the group consisting of hydrogen and methyl.

In another preferred embodiment at least one of R₃ and R₄ is selected from the group consisting of methyl and methoxy.

In another preferred embodiment at least one of R₁ and R₄ is selected from the group consisting of methyl and methoxy.

In another preferred embodiment R₂ is different from hydrogen.

In another preferred embodiment both R₂ and R₄ are different from hydrogen.

In another preferred embodiment R₂ is methyl.

In another preferred embodiment R₂ is methyl and R₄ is different from hydrogen

Combinations of the above-mentioned embodiments do also constitute specific embodiments of the present invention.

The term "pharmaceutically acceptable salts" means salts which are acceptable for administration to a patient, such as a mammal (e.g., salts having acceptable mammalian safety for a given dosage regime). Such salts can be derived from pharmaceutically acceptable inorganic or organic bases and from pharmaceutically acceptable inorganic or organic acids. Salts derived from pharmaceutically acceptable inorganic bases include ammonium, calcium, copper, ferric, ferrous, lithium, magnesium, manganic, manganous, potassium, sodium, zinc and the like. Particularly preferred are ammonium, calcium, magnesium, potassium and sodium salts. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable acids include acetic, ascorbic, benzenesulfonic, benzoic, camphosulfonic, citric, ethanesulfonic, edisylic, fumaric, gentisic, gluconic, glucoronic, glutamic, hippuric, hydrobromic, hydrochloric, isethionic, lactic, lactobionic, maleic, malic, mandelic, methanesulfonic, mucic, naphthalenesulfonic, naphthalene-1,5-disulfonic, naphthalene-2,6-disulfonic, nicotinic, nitric, orotic, pamoic, pantothenic, phosphoric, succinic, sulfuric, tartaric, p-toluenesulfonic, xinafoic and the like. Particularly preferred are citric, hydrobromic, hydrochloric, isethionic, maleic, naphthalene-1,5-disulfonic, phosphoric, sulfuric and tartaric acids.

According to the first aspect of the invention, specific compounds of formula (I) are selected from the group consisting of:
2-((6-amino-3-methoxy-2-methylphenoxy)-3-chloronaphthalene-1,4-dione;
2-((6-amino-2-chloro-3-methoxyphenoxy)-3-chloronaphthalene-1,4-dione;
2-((2-amino-4-chloro-3-methylphenoxy)-3-chloronaphthalene-1,4-dione;
2-((2-amino-3,5-dimethylphenoxy)-3-chloronaphthalene-1,4-dione;
2-((2-amino-4-chloro-6-methylphenoxy)-3-chloronaphthalene-1,4-dione;
2-((2-amino-4-bromo-6-methylphenoxy)-3-chloronaphthalene-1,4-dione;
2-((2-amino-3,4-dimethylphenoxy)-3-chloronaphthalene-1,4-dione;
2-((2-amino-3,5,6-trimethylphenoxy)-3-chloronaphthalene-1,4-dione;
2-((2-amino-3,4,6-trimethylphenoxy)-3-chloronaphthalene-1,4-dione;
and pharmaceutically acceptable salts thereof.

Another aspect of the present invention is to provide a process for preparing the compounds of formula **(I).** The synthesis of compounds of general formula **(I)** is performed by reaction of 2-aminophenols (**III**), wherein R₁, R₂, R₃ and R₄ are as defined for the compounds of formula **(I),** with 2,3-dichloronaphthalene-1,4-dione (**II**), under basic conditions.

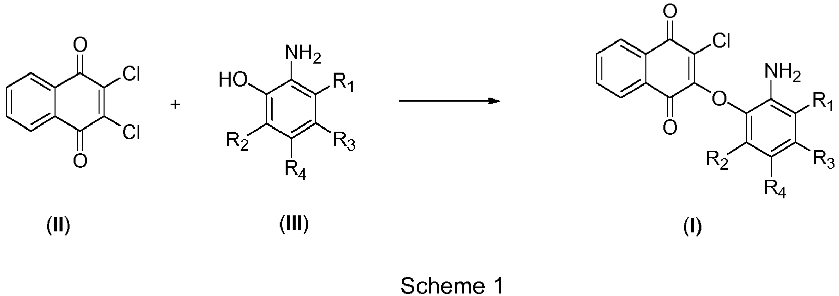

The synthesis involves a single nucleophilic addition of the 2-aminophenols (**III**) by displacement of one of the chlorines in the 2,3-dichloronaphthalene-1,4-dione (**II**). This reaction runs at room temperature until completion in a suitable solvent. Suitable solvents are, for example, polar aprotic solvents such as linear, branched or cyclic ethers (e.g. diethylether, methyl tert-butyl ether, tetrahydrofuran, dioxane, dimethoxyethane), aliphatic or aromatic hydrocarbons and their halogenated derivatives (e.g. heptane, benzene, toluene, chloroform), dimethylsulfoxide, amides (e.g. dimethylformamide, N-methylpyrrolidone, hexamethyl-phosphoric triamide), cyclic ureas (e.g. 1,3-dimethyl-tetrahydro-2[*1H*]-pyrimidinon), acetonitrile or mixtures thereof. The basic conditions can be produced, for example, by the addition of an inorganic base, such as an alkali metal or alkaline earth metal hydroxide, carbonate or bicarbonate, for example sodium, potassium or calcium hydroxide, carbonate or bicarbonate. Useful processes for recovering the resultant compounds of formula (I) include conventional methods known to the person skilled in the art such as crystallization, distillation and chromatographic processes.

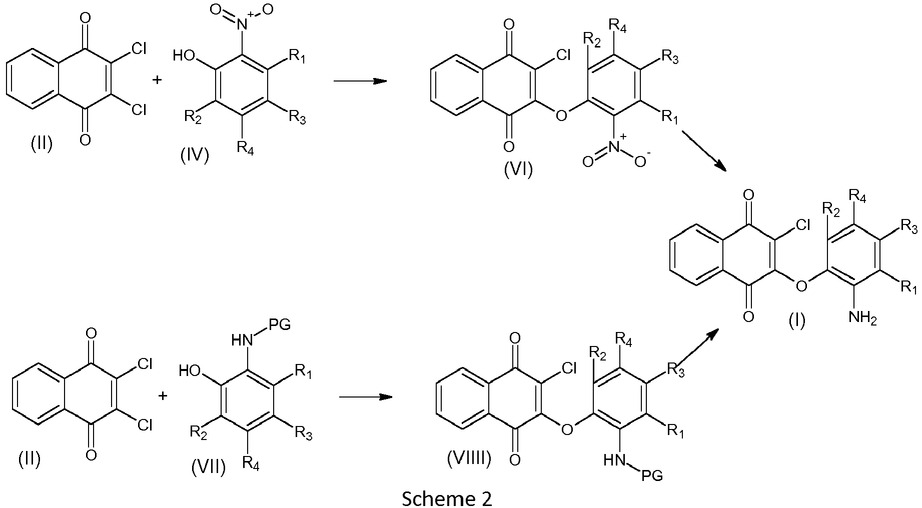

Alternative syntheses involve either the use of 2-nitrophenols (IV) or 2-aminoprotectedphenols (VII). In both cases, an analogous single nucleophilic displacement of one of the chlorines in the 2,3-dichloronaphthalene-1,4-dione (**II**) is observed under the reaction conditions described above. In these alternative syntheses an additional step is required to obtain the final product (I). In the first case, nitroderivatives (VI) are reduced to the amine by standard procedures described elsewhere, for example using tinchloride as reducing agent in a polar solvent. In the case of the amino protected derivatives (VIII), deprotection is performed by standard methods described elsewhere for each specific protecting group, for example trifruoroacetic acid in dichloromethane as solvent in the case of the N-Boc protecting group.Another aspect of the present invention is to provide a pharmaceutical composition containing a compound of formula (I) of the present invention or pharmaceutically acceptable salts, in association with one more pharmaceutically acceptable excipients.

The pharmaceutical compositions include those suitable for oral, rectal and parenteral (including subcutaneous, intramuscular, and intravenous) administration, although the most suitable route will depend on the nature and severity of the condition being treated. The most preferred route of the present invention is the oral route. The compositions may be conveniently presented in unit dosage form, and prepared by any of the methods well known in the art of pharmacy.

The active compound can be combined with pharmaceutical excipients according to conventional pharmaceutical compounding techniques. The excipients may take a wide variety of forms depending on the form of the preparation desired for administration, e.g. oral or parenteral (including intravenous injections or infusions). In preparing the compositions for oral dosage form any of the usual pharmaceutical media may be employed. Usual pharmaceutical media include, for example, water, glycols, oils, alcohols, flavoring agents, preservatives, coloring agents, and the like, in the case of oral liquid preparations (such as for example, suspensions, solutions, emulsions and elixirs); aerosols; or carriers such as starches, sugars, microcrystalline cellulose, diluents, granulating agents, lubricants, binders, disintegrating agents and the like, in the case of oral solid preparations (such as for example, powders, capsules, and tablets) with the oral solid preparations being preferred over the oral liquid preparations.

Because of their ease of administration, tablets and capsules represent the most advantageous oral dosage unit form, in which case solid pharmaceutical carriers are employed. If desired, tablets may be coated by standard aqueous or non-aqueous techniques.

Another aspect of the present invention is to provide the compounds of formula **(I)** of the invention for use as a medicament.

Another aspect of the present invention is to provide the compounds of formula **(I)** of the invention for use as therapeutic active substances for the treatment of conditions mediated by agonizing the orexin 2 receptor. This aspect could be also formulated as the use of the compounds of formula (I) of the invention as defined above for the preparation of a medicament for the treatment of a condition mediated by agonizing the orexin 2 receptor. It also relates to a method for the treatment of a mammal suffering or susceptible to suffer from a condition mediated by agonizing the orexin 2, wherein the method comprises administering to said mammal an effective amount of the compounds of formula (I) of the invention.

Another aspect of the present invention is to provide the compounds of formula **(I)** of the invention for use as therapeutic active substances for the treatment of a condition selected from the group consisting of narcolepsy, excessive daytime sleepiness, nighttime insomnia, depression, sleep apnea and jet lag, for promoting wakefulness and emergence from anesthesia, or for preventing diet-induced obesity. This aspect could be also formulated as the use of the compounds of formula (I) of the invention as defined above for the preparation of a medicament for the treatment of a condition selected from the group consisting of narcolepsy, excessive daytime sleepiness, nighttime insomnia, depression, sleep apnea and jet lag, for promoting wakefulness and emergence from anesthesia, or for preventing diet-induced obesity. It also relates to a method for the treatment of a mammal suffering or susceptible to suffer from a condition selected from the group consisting of narcolepsy, excessive daytime sleepiness, nighttime insomnia, depression, sleep apnea and jet lag, for promoting wakefulness and emergence from anesthesia, or for preventing diet-induced obesity, wherein the method comprises administering to said mammal an effective amount of the compounds of formula (I) of the invention.

A suitable dosage range for use is from about 0.01 mg to about 100.00 mg total daily doses, given as a once daily administration or in divided doses if required.

In accordance with the results obtained, the compounds of formula (I) of the present invention have evidenced pharmacological activity. The pharmacological activity of the compounds of the present invention was determined as described below.

### BIOLOGICAL EVALUATION

### Functional assay for the in vitro evaluation of the OX2 receptor agonist activity

The method of choice for the in vitro evaluation of OX2 receptor agonists is the measure of the increase in intracellular calcium induced by the activation of the receptor. OX2 receptor is stably expressed in a cell line co-expressing apoaequorin, a photoprotein which needs a hydrophobic prosthetic group, coelenterazine, to be converted to aequorin, the active enzyme. After calcium binding, aequorin oxidizes coelenterazine to coelenteramide with the production of CO₂ and emission of light. The measurement of light emission after the addition of agonists reflects its ability to activate the receptor.

### Experimental methodology

### Abbreviations:

FBS: Fetal Bovine serum
DMEM: Dulbecco's Modified Eagle Medium
BSA: Bovine serum albumin

Cell line co-expressing apoaequorin and OX2 receptor were cultured as a monolayer at 37°C and 5% CO₂ in complete Ham's F12 medium supplemented with FBS 10%, penicillin (100 IU/ml)-streptomycin (100 µg/ml), Geneticin (G418, 400 µg/ml) and Zeocin (250 µg/ml). Before starting the experiment, cells were incubated with a medium without antibiotics for 18 hours. After this incubation, the cells were detached from the culture plates and they were resuspended in DMEM-F12/0.1% BSA at a final concentration of 5x10⁶ cells/ml. Cells were then incubated with 5 µM of coelenterazine at room temperature on a vertical rotating wheel for at least 3 hours. Following incubation with coelenterazine cells were diluted 1:10 with DMEM-F12/0.1% BSA and incubated in magnetic agitation for 1 hour.

Dose-response curves were performed for each compound. 50 µl of pre-loaded cells were injected over test compounds in a 96 wells plate. Results were expressed as the concentration of the compound where 50% of its maximal effect is observed (EC₅₀), related to the maximum effect induced by the orexin peptide on the receptor. The results are summarized in table 1

**Table 1. OX2R Agonism**

| | | | | | |
|---|---|---|---|---|---|
| 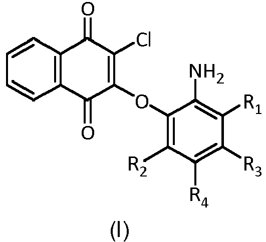 | | | | | |

| ***Example*** | ***R₁*** | ***R₂*** | ***R₃*** | ***R₄*** | ***EC₅₀ (nM)*** |
|---|---|---|---|---|---|
| 1 | H | Me | H | MeO | 28 |
| 2 | H | Cl | H | MeO | 50 |
| 3 | Me | H | Cl | H | 110 |
| 4 | Me | H | H | Me | 60 |
| 5 | H | Me | Cl | H | 80 |
| 6 | H | Me | Br | H | 90 |
| 7 | Me | H | Me | H | 50 |
| 8 | Me | Me | H | Me | 58 |
| 9 | Me | Me | Me | H | 130 |

### COMPARATIVE EXAMPLES

Using the same experimental methodology described above, the EC₅₀ of compounds described in PCT/EP2014/062349 is reported below.

**Table 2. OX2R Agonism**

| | | | | | |
|---|---|---|---|---|---|
| 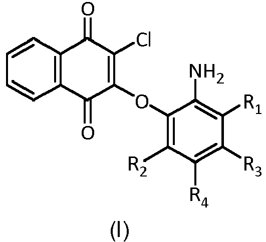 | | | | | |

| ***Example*** | ***R₁*** | ***R₂*** | ***R₃*** | ***R₄*** | ***EC₅₀ (nM)*** |
|---|---|---|---|---|---|
| C1 | H | H | H | H | 516 |
| C2 | H | H | Me | H | 242 |
| C3 | H | H | Cl | H | 737 |
| C4 | H | H | H | Cl | 747 |
| C5 | H | H | F | H | 578 |
| C6 | H | H | CO₂Me | H | 4057 |
| C7 | H | H | COMe | H | 4370 |
| C8 | H | H | CF₃ | H | 700 |
| C9 | H | H | H | F | 390 |
| C10 | H | H | Me | Me | 670 |
| C11 | H | Cl | Cl | H | 390 |
| C12 | Me | Me | H | H | 150 |

### PREPARATIVE EXAMPLES

### Analytical Data

LC-MS analyses were performed using two different HLPC methods noted as Met1 or Met2 in the tables of examples. These methods are described as follows:
Method 1: Column Polaris C-18 (50 mm x 4.6 mm), temperature 30°C, flow 1ml/min, solvent A = acetonitrile + 0.05% formic acid, B = formic acid water solution 0.05%, gradient: t = 0min 10%A; t = 9min 90% A.
Method 2: Column Axquity UPLC BEH C18 (1.7 um 2.1 x 100 mm), temperature 40°C, flow 0.5ml/min, solvent A = acetonitrile, B = NH₄HCO₃ 10 mM, gradient: t = 0min 10% A; t=0.25min 10% A; t = 3min 90% A; t=3.75min 90% A; t=3.76min 10% A; t=5min 10% A.

### Example 1

### 2-(6-amino-3-methoxy-2-methylphenoxy)-3-chloronaphthalene-1,4-dione

6-amino-3-methoxy-2-Methyl phenol (202 mg, 1.32 mmol) and sodium carbonate (70 mg, 0.66 mmol) were added sequentially to a solution of 2,3-dichloro-1,4-naphtoquinone (300 mg, 0.66 mmol) in dimethylsulfoxide (5 mL). The reaction was then stirred at 20°C until completion. Once the reaction was finished, water was added and the reaction crude was then acidified to pH= 5-6 with HCl 1 N aqueous solution. Then it was extracted with ethyl acetate and the organic layer was washed twice with water. The product was finally purified by column chromatography, obtaining 80 mg of the title product (yield = 17%).
LC-MS (method 1): Retention time (min) = 12.58; m/z = 343.8 [M+H]⁺

### Examples 2 to 9 and comparative examples C1 to C12

Following an analogous procedure to the synthesis of example 1, examples 2-9 were obtained by reacting the appropriate 2,3-dichloro-1,4-naphtoquinone compound with the appropriate 2-aminophenol compound. IUPAC names, yields and analytical data are summarized in table 2.

Comparative examples C1 to C12 may also be prepared following an analogous procedure to the synthesis of example 1 by reacting the appropriate 2,3-dichloro-1,4-naphtoquinone compound with the appropriate 2-aminophenol compound.

**Table 2. IUPAC Names, Yields (%), Retention times (min), HPLC Methods and MH⁺ (m/z) values**

| **Ex.** | ***IUPAC Name*** | % | ***min*** | ***HPLC*** | ***m*/*z*** |
|---|---|---|---|---|---|
| 2 | 2-(6-amino-2-chloro-3-methoxyphenoxy)-3-chloronaphthalene-1,4-dione | 33 | 4.79 | Met1 | 364.2 |
| 3 | 2-(2-amino-4-chloro-3-methylphenoxy)-3-chloronaphthalene-1,4-dione | 49 | 2.40 | Met2 | 348.2 |
| 4 | 2-(2-amino-3,5-dimethylphenoxy)-3-chloronaphthalene-1,4-dione | 4 | 2.35 | Met2 | 327.8 |
| 5 | 2-(2-amino-4-chloro-6-methylphenoxy)-3-chloronaphthalene-1,4-dione | 5 | 2.26 | Met2 | 348.2 |
| 6 | 2-(2-amino-4-bromo-6-methylphenoxy)-3-chloronaphthalene-1,4-dione | 1 | 1.68 | Met2 | 392.6 |
| 7 | 2-(2-amino-3,4-dimethylphenoxy)-3-chloronaphthalene-1,4-dione | 26 | 2.34 | Met2 | 327.8 |
| 8 | 2-(2-amino-3,5,6-trimethylphenoxy)-3-chloronaphthalene-1,4-dione | 27 | 5.49 | Met1 | 341.8 |
| 9 | 2-(2-amino-3,4,6-trimethylphenoxy)-3-chloronaphthalene-1,4-dione | 10 | 5.52 | Met1 | 341.8 |

## Claims

1. A compound of formula (I): 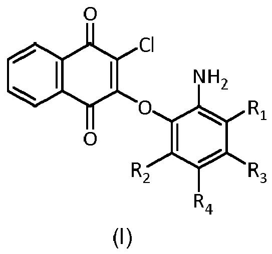 wherein:
R₁ is selected from the group consisting of hydrogen, chlorine, bromine and C₁₋₃ alkyl and R₂ is selected from the group consisting of hydrogen, chlorine and C₁₋₃ alkyl with the condition that at least one of R₁ and R₂ is different from hydrogen;
R₃ is selected from the group consisting of hydrogen, halogen and C₁₋₃ alkyl and R₄ is selected from the group consisting of hydrogen, chlorine, bromine, C₁₋₃ alkoxy and C₁₋₃ alkyl with the condition that at least one of R₃ and R₄ is different from hydrogen;
with the proviso that R₂ and R₃ are not simultaneously a chlorine atom and pharmaceutically acceptable salts thereof.

2. A compound according to claim 1, wherein:
R₁ is selected from the group consisting of hydrogen and methyl and R₂ is selected from the group consisting of hydrogen, chlorine and methyl with the condition that at least one of R₁ and R₂ is different from hydrogen;
R₃ is selected from the group consisting of hydrogen, bromine, chlorine and methyl and R₄ is selected from the group consisting of hydrogen, methoxy and methyl with the condition that at least one of R₃ and R₄ is different from hydrogen;
with the proviso that R₂ and R₃ are not simultaneously a chlorine atom and pharmaceutically acceptable salts thereof.

3. A compound according to claim 2, wherein R₃ is selected from the group consisting of hydrogen and methyl.

4. A compound according to any one of the preceding claims, wherein at least one of R₃ and R₄ is selected from the group consisting of methyl and methoxy.

5. A compound according to any one of the preceding claims, wherein at least one of R₁ and R₄ is selected from the group consisting of methyl and methoxy.

6. A compound according to any one of the preceding claims, wherein R₂ is different from hydrogen.

7. A compound according to claim 6, wherein R₂ is methyl.

8. A compound according to any one of claims 6 to 7, wherein R₄ is different from hydrogen.

9. A compound according to claim 1, wherein the compound is selected from the group consisting of:
2-((6-amino-3-methoxy-2-methylphenoxy)-3-chloronaphthalene-1,4-dione;
2-((6-amino-2-chloro-3-methoxyphenoxy)-3-chloronaphthalene-1,4-dione;
2-((2-amino-4-chloro-3-methylphenoxy)-3-chloronaphthalene-1,4-dione;
2-((2-amino-3,5-dimethylphenoxy)-3-chloronaphthalene-1,4-dione;
2-((2-amino-4-chloro-6-methylphenoxy)-3-chloronaphthalene-1,4-dione;
2-((2-amino-4-bromo-6-methylphenoxy)-3-chloronaphthalene-1,4-dione;
2-((2-amino-3,4-dimethylphenoxy)-3-chloronaphthalene-1,4-dione;
2-((2-amino-3,5,6-trimethylphenoxy)-3-chloronaphthalene-1,4-dione;
2-((2-amino-3,4,6-trimethylphenoxy)-3-chloronaphthalene-1,4-dione.

10. A process for the preparation of a compound of formula **(I)** as defined in any of claims 1-9, comprising:
reacting 2,3-dichloronaphthalene-1,4-dione of formula (**II**) 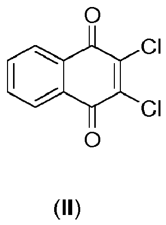 with a 2-aminophenol of formula (**III**) 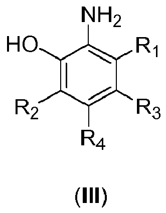
wherein R₁, R₂, R₃ and R₄ are as defined for the compound of formula **(I),** under basic conditions.

11. A pharmaceutical composition comprising a compound of formula **(I)** according to any of claims 1-9 and at least one pharmaceutically acceptable excipient.

12. A compound as defined in any of claims 1-9 for use as a medicament.

13. A compound as defined in any of claims 1-9 for use as a therapeutic active substance for the treatment of conditions mediated by agonizing the orexin 2 receptor.

14. A compound as defined in any of claims 1-9 for use as a therapeutic active substance for the treatment of a condition selected from the group consisting of narcolepsy, excessive daytime sleepiness, nighttime insomnia, depression, sleep apnea and jet lag, for promoting wakefulness and emergence from anesthesia, or for preventing diet-induced obesity.

15. Use of a compound as defined in any of claims 1-9 for the preparation of a medicament for the treatment of a condition mediated by agonizing the orexin 2 receptor.

16. Use of a compound as defined in any of claims 1-9 for the manufacture of a medicament for the treatment of a condition selected from the group consisting of narcolepsy, excessive daytime sleepiness, nighttime insomnia, depression, sleep apnea and jet lag, for promoting wakefulness and emergence from anesthesia, or for preventing diet-induced obesity.
